# EUROPEAN PATENT SPECIFICATION

(11) **EP 2 197 423 B1**
(45) Date of publication and mention of the grant of the patent: **03.12.2014**
(21) Application number: 07819025.3
(22) Date of filing: 16.10.2007
(51) Int. Cl.: A61K 9/16, A61K 31/4535, A61K 9/26, A61K 9/36, A61K 9/48

(54) **IMPROVED PHARMACEUTICAL COMPOSITION CONTAINING A SELECTIVE ESTROGEN RECEPTOR MODULATOR AND METHOD FOR THE PREPARATION THEREOF**
VERBESSERTE PHARMAZEUTISCHE ZUSAMMENSETZUNG MIT EINEM SELEKTIVEN ÖSTROGEN-REZEPTOR-MODULATOR UND VERFAHREN ZU SEINER HERSTELLUNG
COMPOSITION PHARMACEUTIQUE AMÉLIORÉE CONTENANT UN MODULATEUR DE RÉCEPTEUR STROGÈNE SÉLECTIF, ET PROCÉDÉ DE PRÉPARATION DE CELUI-CI

(43) Date of publication of application: 23.06.2010
(73) Proprietor: Pharmathen S.A., Pallini Attikis 15351 (GR)
(72) Inventor: KARAVAS, Evangelos, GR-153 51 Pallini Attikis (GR); KOUTRIS, Efthimios, GR-153 51 Pallini Attikis (GR); BIKIARIS, Dimitrios, GR-543 51 Thessaloniki (GR); SAMARA, Vicky, GR-153 51 Pallini Attikis (GR); KALASKANI, Anastasia, GR-153 51 Pallini Attikis (GR); STATHAKI, Eleni, Gr-153 51 Attikis (GR)
(86) International application number: PCT/EP2007/008955
(87) International publication number: WO 2009/049643

(56) References cited:
- EP-A- 0 670 162
- WO-A-02/03987
- US-A1- 2006 068 010

## Description

### TECHNICAL FIELD OF THE INVENTION

The present invention relates to improved dosage forms such as tablets and capsules and in particular to a formulation for oral administration comprising a therapeutically effective quantity of a selective estrogen receptor modulator, and more particularly Raloxifene, its esters or ethers or a pharmaceutically acceptable salt or derivative thereof, in combination with a super disintegrant, such as sodium starch glycolate as a dissolution enhancing agent and a method for the preparation thereof.

### BACKGROUND OF THE INVENTION

Osteoporosis constitutes a systemic skeletal disease characterized by low bone density and deterioration of bone tissue. The consequence of this disease is a significant increase in bone fragility, resulting to an increased susceptibility to fractures. The risk of osteoporosis is significantly increased with ageing of the population, and furthermore, women are at greater risk of osteoporosis than men. In women, bone loss is accelerated during the period following menopause.

Treatment for osteoporosis is concentrated on slowing down or preventing bone loss and on control of pain associated with the disease. There are different types of medicines used to treat or prevent osteoporosis, including Raloxifene, bisphosphonates, hormone replacement therapy and calcitonin. There are also several ways to prevent osteoporosis, such as regular exercise and an adequate dietary intake of calcium, vitamin C & D and protein.

Raloxifene constitutes an oral Selective Estrogen Receptor Modulator (SERM) that belongs to the benzothiophene compounds and it is used as the active pharmaceutical ingredient in compositions used for the prevention or treatment of osteoporosis in postmenopausal women.

Menopause is a condition characterized by an imbalance between bone destruction and bone formation, which is importantly related to progressive loss of estrogen. This imbalance may also be associated with age-related impairment of osteoblasts or their precursors.

The term "Selective Estrogen Receptor Modulators" (SERMs), such as Raloxifene, refers to estrogen-like drugs, also known as "designer estrogens". This means that Raloxifene possesses some, but not all, of the actions of estrogen. Raloxifene has been classified as a SERM, because it prevents bone loss (like estrogen) and lowers serum cholesterol (like estrogen), but it does not stimulate the uterus. Thus, Raloxifene acts like an estrogen by stopping the progression of bone loss that is developed in women after menopause.

In fact, Raloxifene acts by reducing the bone resorption, resulting to a decrease in the overall bone turnover. This action of Raloxifene is because of its binding to estrogen receptors, regulating estrogen-regulated gene expression by at least two distinct pathways which are ligand-, tissue-, and/or gene-specific.

Clinical studies have indicated that Raloxifene has estrogen-agonistic effects on bone (increase in bone density) and on lipid (decrease in total and LDL cholesterol levels) metabolism and estrogen-antagonistic effects on uterine endometrium and breast tissue. This data denote that Raloxifene presents high tissue selectivity, causing fewer adverse effects than typical estrogen therapy.

A particularly useful pharmaceutically acceptable salt of Raloxifene is the hydrochloride salt, which can be easily prepared by reacting hydrogen chloride with a solution of Raloxifene in an organic solvent, such as tetrahydrofuran or methanol.

Raloxifene hydrochloride is chemically designated as [6-hydroxy-2-(4-hydroxyphenyl) benzothiophen-3-yl]-[4-[2-(1-piperidinyl) ethoxy] phenyl] methanone hydrochloride and has the empirical formula of C₂₈ H₂₇ NO₄ S·HCl. Its molecular weight is 510.05 g/mol, and 473.584g/mol as a free base.

The hydrochloride salt of Raloxifene has a very poor solubility in aqueous media, limiting therefore its bioavailability. In addition, Raloxifene hydrochloride constitutes a highly hydrophobic drug, fact that constitutes a significant barrier for its absorption.

Furthermore, the stability, the bioavailability and the release rate of pharmaceutical compositions containing a selective estrogen receptor modulator and in particular, Raloxifene or salt thereof can also be influenced by the selection of the excipients.

Various methods are already known for the industrial preparation of oral dosage forms comprising a selective estrogen receptor modulator e.g. Raloxifene or salt thereof, as an active ingredient due to its useful therapeutical properties. However, the prior art has encountered substantial difficulties in the production of the oral solid formulations of a desirable bioavailability due to the very poor solubility of said active ingredient.

EP 0 670 162 discloses a pharmaceutical composition with increased solubility in aqueous media, which comprises Raloxifene hydrochloride, in combination with a surfactant, a water-soluble diluent and a hydrophilic binder.

WO 2005/04917 discloses a pharmaceutical formulation with enhanced solubility, which comprises a hydrophobic drug in particularly Raloxifene HCL and polyethylene glycol (PEG), wherein the ratio of PEG to drug by weight is from about 0.2: 1 to about 10: 1.

Furthermore, US 2006/099252 discloses an oral solid pharmaceutical formulation with increased dissolution rate, which comprises an active agent with low aqueous solubility, particularly Raloxifene HCl, combined with starch at a concentration of greater than 25% w/w.

Although each of the above patents represents an attempt to overcome the low aqueous solubility and bioavailability problems associated with pharmaceuticals compositions comprising a selective estrogen receptor modulator, there still exists a need for improving the solubility of such pharmaceutical compositions.

### SUMMARY OF THE INVENTION

It is, therefore, an object of the present invention to provide an improved solid dosage formulation for oral administration containing a selective estrogen receptor modulator, and in particular Raloxifene or salt thereof as an active ingredient, which overcomes the deficiencies of the prior art and increases the dissolution rate of the active ingredient.

Another aspect of the present invention is to provide a solid dosage formulation for oral administration containing a selective estrogen receptor modulator, and in particular Raloxifene or salt thereof as an active ingredient, which is bioavailable and effective with sufficient self-life and good pharmacotechnical properties.

Moreover, another aspect of the present invention is to provide a solid dosage formulation for oral administration containing a selective estrogen receptor modulator, and in particular Raloxifene or salt thereof as an active ingredient, which can be prepared in dosage forms of different strength by proportionally adjusting the quantities of the excipients and the active ingredient, thereby providing a pharmacotechnical linearity, without affecting the dissolution profile and bioavailability of the active ingredient.

A further aspect of the present invention is to provide a method for the preparation of a stable solid dosage formulation for oral administration containing a selective estrogen receptor modulator, and in particular Raloxifene or salt thereof as an active ingredient, permitting an enhanced release of the active medicament and used in the prevention and treatment of osteoporosis in postmenopausal women with improved pharmacotechnical characteristics of the composition.

In accordance with the above objects of the present invention, a pharmaceutical composition for oral administration is provided comprising a selective estrogen receptor modulator, and in particular Raloxifene, its esters or ethers or a pharmaceutically acceptable salt or derivative thereof as an active ingredient, and an effective amount of a super disintegrant such as sodium starch glycolate as an agent to enhance bioavailability and to increase the drug release.

According to another embodiment of the present invention, a process for the preparation of solid dosage forms for oral administration such as tablets, capsules and sachets, containing a selective estrogen receptor modulator; and in particular Raloxifene, its esters or ethers or a pharmaceutically acceptable salt or derivative thereof as an active ingredient is provided, which comprises:
- Forming a first homogenous blend by mixing the total quantity of said active ingredient with a total quantity of super disintegrant such as sodium starch glycolate as a dissolution enhancing agent to facilitate the drug release and increase bioavailability, and optionally a pH adjusting agent such as citric acid monohydrate;
- dissolving the total quantity of a surfactant such as Poloxamer 407 into purified water;
- adding the first blend into the formed solution;
- adding to the above kneaded mixture the total quantity of at least one optional excipient such as a binder, a disintegrant, a lubricant, a colorant and/or a glidant until uniform and wet granulating;
- drying the wet mass;
- sieving the dried material to achieve the desired granule size
- adding and mixing the dried material with at least one optional excipient of the external phase such as a binder, a disintegrant, a lubricant, a colorant and/or a glidant until uniform, and
- formulating the resulting mixture in a solid dosage form either by compressing it into a desired tablet form or by filling capsules or sachets.

Further preferred embodiments of the present invention are defined in dependent claims 2 to 10 and 12 to 13.

Other objects and advantages of the present invention will become apparent to those skilled in the art in view of the following detailed description.

### BRIEF DESCRIPTION OF THE DRAWINGS

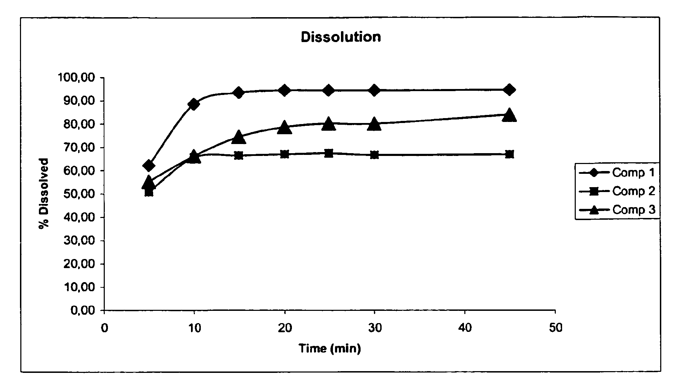

Fig. 1 shows a dissolution diagram of the Raloxifene HCL compositions of examples 1, 2 and 3 according to the present invention.

### DETAILED DESCRIPTION OF THE INVENTION

For the purposes of the present invention, a pharmaceutical composition comprising an active ingredient (a selective estrogen receptor modulator, and in particular Raloxifene or salt thereof) is considered to have "low aqueous solubility" if said ingredient dissolves less or more slowly than it does on its own and/or in known pharmaceutical compositions.

The term "pharmaceutically acceptable salt" refers to a salt that is not toxic at the specific therapeutic dosage and to a salt that does not independently possess significant pharmacological activity.

An excipient is considered to be "incompatible" with an active ingredient (a selective estrogen receptor modulator, and in particular Raloxifene or salt thereof) if it inhibits the dissolution rate of said active ingredient, that is to say, if said active ingredient (a selective estrogen receptor modulator, and in particular Raloxifene or salt thereof) dissolves less or slower in the presence of said excipient when compared with the dissolution rate of said active ingredient (a selective estrogen receptor modulator, and in particular Raloxifene or salt thereof) on its own. The terms "incompatibility", "compatible" and "compatibility" are defined accordingly.

The active ingredient (a selective estrogen receptor modulator, and in particular Raloxifene or salt thereof) contained in a dosage form is "bioavailable", if when administered in a dosage form is released from the dosage form, absorbed and reaches, at least the same, concentration levels in plasma as any of the marketed products containing the same quantity of the same active ingredient and intended for the same use.

Although the pharmaceutical composition may be in various forms, the preferred solid forms are tablets, capsules and caplets.

The improved solid pharmaceutical composition of the present invention is characterized by physicochemical properties suitable for the tablet formulation by wet granulation, the adequate release rate of the active ingredient (a selective estrogen receptor modulator, and in particular Raloxifene or salt thereof) and the storage stability, by employing excipients practically devoid the tendency of the active ingredient to degradation in humidity or ambient temperature.

As already mentioned certain selective estrogen receptor modulators have low aqueous solubility and their tendency gets stronger when they are formulated and mixed with excipients or other active substances.

It has been surprisingly found that the object of the present invention is achieved by employing a super disintegrant such as sodium starch glycolate, also called Primojel, as a dissolution enhancing agent.

Sodium starch glycolate, a representative example of a cross-linked starch, is a modified starch possessing very significant disintegrating properties, and is practically insoluble in organic solvents. Chemically, Primojel constitutes a low substituted carboxy methyl starch.

Sodium starch glycolate presents very good hydration capacity and very good flow properties in comparison to other super disintegrants. Further, it presents the tendency to absorb water rapidly, so it swells in a significant amount. Therefore, this rapid water absorption by sodium starch glycolate molecules has as a result a significant increase in the volume of granules resulting to rapid and uniform disintegration.

Sodium starch glycolate incorporated in a pharmaceutical composition facilitates the breakup or disintegration of the content of the tablet into smaller particles that dissolve more rapidly than in the absence of disintegrating agents.
Moreover, it presents the benefit to counterbalance the efficiency of the tablet binder, as well as the physical forces that develop during the compression procedure for the manufacture of a tablet.

Sodium starch glycolate is incorporated into the composition of the present invention by internal addition (intragranular). During the internal addition method, the disintegrating agent is mixed with other powders prior to wetting the powder mixture with the granulating fluid. The result of this method is that the super disintegrant becomes incorporated within the granules of the composition, resulting to a more effective disintegration process.

Moreover, one of the main disadvantages of the selective estrogen receptor modulator, and in particular Raloxifene HCL, is the fact that, they are very labile to acidic pH environment, and consequently many limitations concerning the choice of excipients are raised.

The lubricant should be very carefully selected because some of them are very hydrophobic and affect negatively the disintegration and dissolution while it has been shown to cause bioavailability problems. The manufacturing process should also be very carefully determined because relatively high concentrations of lubricant and/or glidant reduce crashing strength and increase disintegration time especially when associated with prolonged mixing times.

In addition, the pH of the composition of the present invention was adjusted to 6.0 using aqueous citric acid as needed. The molecule of citric acid has three carboxyl groups, each of which loses a proton in a solution. Therefore, citrate ions are formed, which are excellent buffers for controlling the PH of acidic solutions.

Moreover, any excipient may optionally be added to the above composition, provided that they are compatible with the active ingredient of the composition, in order to overcome problems associated with unfavorable pharmacotechnical characteristics of these substances, and in order to increase the stability of the drug and the self-life of the pharmaceutical product, and provide a product exhibiting excellent bioavailability.

The present invention can be applied in the formulation of tablets, capsules, caplets, sachets or other solid dosage forms for oral or sub-lingual administration of an active ingredient having solubility and bioavailability problems.

Furthermore, it is possible to prepare dosage forms of different strength using appropriate quantity of the same composition, thereby limiting the cost of production and minimizing the number, and consequently the cost, of clinical studies required for the approval of the product by the authorities.

Therefore, in a first embodiment, the present invention provides a pharmaceutical composition comprising from about 0.5% to30% by weight of Raloxifene or salt thereof and from about 3% to 30% by weight of sodium starch glycolate. The weight ratio of Raloxifene or salt thereof to sodium starch glycolate is preferably 10:1 to 1:60.

More preferred pharmaceutical compositions according to the present invention comprise approximately 3% to 30%, more preferably 5% to 25% and most preferably 7% to 20% by weight of sodium starch glycolate.

The preferred pharmaceutical compositions are in the form of solid dosage forms for oral or sub-lingual administration such as tablets, capsules, caplets, troches, pastilles, pills, lozenges and the like, in all shapes and sizes, coated or uncoated.

All percentages stated herein are weight percentages based on total composition weight, unless otherwise stated.

Another embodiment of the present invention is the use of the wet granulation process for the preparation of solid dosage forms for oral administration such as tablets, capsules and sachets containing selective estrogen receptor modulator such as Raloxifene, its esters or ethers or a pharmaceutically acceptable salt or derivative thereof as an active ingredient is provided, which comprises:
- Forming a first homogenous blend by mixing the total quantity of said active ingredient with a total quantity of super disintegrant such as sodium starch glycolate as a dissolution enhancing agent to facilitate the drug release and increase bioavailability, and optionally a pH adjusting agent such as citric acid monohydrate;
- dissolving the total quantity of a surfactant such as Poloxamer 407 into purified water;
- adding the first blend into the formed solution;
- adding to the above kneaded mixture the total quantity of at least one optional excipient such as a binder, a disintegrant, a lubricant, a colorant and/or a glidant until uniform and wet granulating;
- drying the wet mass;
- sieving the dried material to achieve the desired granule size
- adding and mixing the dried material with at least one optional excipient of the external phase such as a binder, a surfactant, a diluent, a disintegrant, a lubricant, a colorant and/or a glidant until uniform, and
- formulating the resulting mixture in a solid dosage form either by compressing it into a desired tablet form or by filling capsules or sachets.

The pharmaceutical compositions of the present invention may also contain one or more additional formulation ingredients selected from a wide variety of excipients. According to the desired properties of the composition, any number of ingredients may be selected, alone or in combination, based upon their known uses in preparation of solid dosage form compositions.

Such ingredients include, but are not limited to, diluents, binders, compression aids, disintegrants, surfactants, wetting agents, glidants, lubricants, flavours, water scavengers, colorants, sweetener, coating agents and preservatives.

The optional excipients must be compatible with the selective estrogen receptor modulator or the salt thereof so that it does not interfere with it in the composition.
Diluents may be, for example, calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol.
Binders may be, for example, acacia mucilage, alginic acid, carbomer, carboxymethylcellulose calcium, carboxymethylcellulose sodium, microcrystalline cellulose, powdered cellulose, ethyl cellulose, gelatin, liquid glucose, guar gum, hydroxyethyl cellulose, hydroxypropyl cellulose, hydroxypropylmethyl cellulose, maltodextrin, methylcellulose, polydextrose, polyethylene oxide, povidone, sodium alginate, starch paste, pregelatinized starch, and sucrose.
Disintegrants may be, for example, alginic acid, carbon dioxide, carboxymethylcellulose calcium, carboxymethylcellulose sodium, microcrystalline cellulose, powdered cellulose, croscarmellose sodium, crospovidone, sodium docusate, guar gum, hydroxypropyl cellulose, methylcellulose, polacrilin potassium, poloxamer, povidone, sodium alginate, sodium glycine carbonate, sodium lauryl sulfate, starch, pregelatinized starch. Surfactants may be, for example, poloxamer, pluronic, ethoxylated castor oil, polyglycolyzed glycerides, acetylated monoglycerides, sorbitan fatty acid esters, polyoxyethylene sorbitan fatty acid esters, monoglycerides or ethoxylated derivatives thereof, diglycerides or polyoxyethylene derivatives thereof, as well as sodium docusate or sodium lauryl sulphate.
Glidants may be, for example, calcium silicate, powdered cellulose, starch, talc, colloidal silicon dioxide.
Lubricants may be e.g. magnesium stearate, polyethylene glycol 4000, polyethylene glycol 6000, sodium lauryl sulfate, starch, talc.

The following examples illustrate preferred embodiments in accordance with the present invention without limiting the scope or spirit of the invention:

### EXAMPLES

### Example 1:Tablet of 60 mg Raloxifene HCL(Comp. 1)

| Ingredients | Percentage by weight (% w/w) | Tablet weight (Mg/tablet) |
|---|---|---|
| **Internal Phase** | | |
| Raloxifene HCl | 24,00 | 60,0 |
| Sodium Starch Glycolate (Primogel) | 12,00 | 30,0 |
| Citric acid | 2,00 | 5,0 |
| Dibasic Calcium Phosphate | 42,00 | 105,0 |
| Microcrystalline Cellulose | 16,70 | 41,75 |
| Poloxamer 407 | 2,50 | 6,25 |
| Purified water | | 125,0 |

| **External Phase** | | |
|---|---|---|
| Magnesium Stearate | 0,80 | 2,0 |
| **Total weight for uncoated tablet** | 100,00 | 250,0 |
| Opadry OY-LS-28908 (II WHITE) | | 7,50 |
| Purified water | | 7,50 |
| Ethyl alcohol | | 67,50 |
| **Total weight for coated tablet** | | 257,50 |

Tablets of the above formulation were prepared according to the following manufacturing process: Raloxifene HCL, sodium starch glycolate and citric acid monohydrate were admixed to complete homogeneity. The total amount of Poloxamer 407 was dissolved in the purified water and is stirred for an adequate period of time till it is completely dissolved. The first blend was kneaded with the kneading solution described above till a homogenous granular mass is produced. Subsequently, the total amount of microcrystalline cellulose and the total amount of dibasic calcium phosphate were added in the above mixture and mixed until a homogenous granular mass was produced. The wetted mass was then dried, passed through a sieve to achieve the desired granule size and further mixed with Mg stearate. Finally, it was formulated in a solid dosage form by compressing it into a desired tablet form. Subsequently, the tablets were film-coated with Opadry II white.

The produced tablets were tested for content uniformity, disintegration, water content and dissolution proving that they are meeting the specifications.

### Example 2:Tablet of 60 mg Raloxifene HCL (Comp. 2)

| Ingredients | Percentage by weight (% w/w) | Tablet weight (Mg/tablet) |
|---|---|---|
| **Internal Phase** | | |
| Raloxifene HCl | 24,00 | 60,0 |
| Sodium Starch Glycolate (Primojel) | 12,00 | 30,0 |
| Dibasic Calcium Phosphate | 44,00 | 110,0 |
| Microcrystalline Cellulose | 16,70 | 41,75 |
| Poloxamer 407 | 2,50 | 6,25 |
| Purified water | | 125,0 |

| **External Phase** | | |
|---|---|---|
| Magnesium Stearate | 0,80 | 2,0 |
| **Total weight for uncoated tablet** | 100,00 | 250,0 |
| Opadry OY-LS-28908 (II WHITE) | | 7,50 |
| Purified water | | 7,50 |
| Ethyl alcohol | | 67,50 |
| **Total weight for coated tablet** | | 257,50 |

Tablets of the composition 2 of Example 2 were prepared according to the manufacturing process used in Example 1.

### Example 3:Tablet of 60 mg Raloxifene HCL (Comp. 3)

| Ingredients | Percentage by weight (% w/w) | Tablet weight (Mg/tablet) |
|---|---|---|
| **Internal Phase** | | |
| Raloxifene HCl | 24,00 | 60,0 |
| Sodium Starch Glycolate | 06,00 | 15,0 |
| Citric acid | 2,00 | 5,0 |
| Dibasic Calcium Phosphate | 42,00 | 105,0 |
| Microcrystalline Cellulose | 22,70 | 56,75 |
| Poloxamer 407 | 2,50 | 6,25 |
| Purified water | | 125,0 |

| **External Phase** | | |
|---|---|---|
| Magnesium Stearate | 0,80 | 2,0 |
| **Total weight for uncoated tablet** | 100,00 | 250,0 |
| Opadry OY-LS-28908 (II WHITE) | | 7,50 |
| Purified water | | 7,50 |
| Ethyl alcohol | | 67,50 |
| **Total weight for coated tablet** | | 257,50 |

Tablets of the composition 3 of Example 3 were prepared according to the manufacturing process used in Example 1.

One of the most critical pharmacotechnical tests is the dissolution test as it is strongly correlated with the bioavailability of the product. For the dissolution method an Apparatus II (paddles) was run at 75rpm, 37°C ± 0.5 °C, for 30min, while as dissolution medium 500ml of HCl 0.01N was used.

Dissolution rate results for each composition tested are given in Table 1. The results show that composition 1 is completely dissolved in about 30 minutes; however composition 2, which does not comprise citric acid and composition 3 with less amount of sodium starch glycolate are not completely dissolved in about 30 minutes.

**TABLE 1:Dissolution profiles of the compositions of Examples 1, 2 and 3**

| **Time (min)** | **Composition 1** | **Composition 2** | **Composition 3** |
|---|---|---|---|
| **5** | 62,21 | 50,83 | 55,24 |
| **10** | 88,62 | 65,65 | 66,25 |
| **15** | 93,61 | 66,54 | 74,59 |
| **20** | 94,55 | 67,09 | 78,78 |
| **25** | 94,52 | 67,48 | 80,33 |
| **30** | 94,40 | 66,67 | 80,07 |
| **45** | 94,62 | 66,93 | 83,94 |

Another object of the present invention was to prepare a pharmaceutical composition that is stable, said active ingredient does not degradates and remains stable for a long period of storage time. For this reason, prior to the clinical trials, composition 1 that exhibits the desirable dissolution profile, was packed in PVC/PE/PVDC Aluminum blisters and exposed to normal (25°C±2°C/60%±5% RH), intermediate (30°C±2°C/65%±5% RH) and accelerated (40°C±2°C/75%±5% RH) stability studies according to the current ICH guidelines. The stability results after twelve months (up to six months for accelerated conditions) are shown in table 2 below.

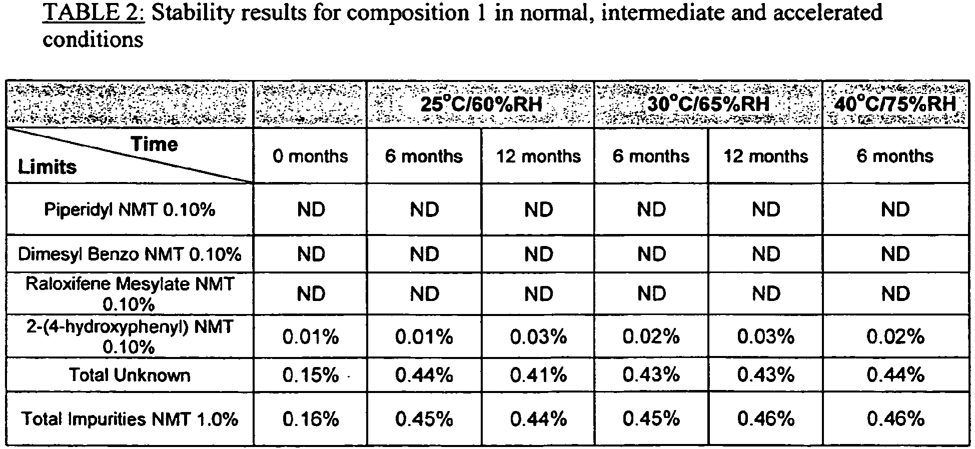

The results demonstrate that a very stable product is produced according to the method described in the present invention.

The composition 1 described above was investigated for its scalability, while a process validation was performed in order to prove the repeatability and accuracy of the manufacturing process and the proposed formulation.

The validation process showed that the composition and the manufacturing process are suitable in order to provide a repeatable and high quality product.

The results show a good stability of the product and compatibility between the drug substance and the excipients proposed by the present invention. The excellent results regarding the physicochemical characteristics, the excellent stability of the product as well as the simple and economic manufacturing process indicate the advantages of the present invention relative to the commonly used methods and excipients for the formulation of Raloxifene HCL.

Another object of the present invention is to manufacture a pharmaceutical dosage form that is "bioavailable", meaning that when administered is released from the dosage form, absorbed and reaches, at least the same, concentration levels in plasma as any of the marketed products containing the same quantity of the same active ingredient and intended for the same use.

The bioavailability and pharmacokinetic profile of composition 1 of the present invention was determined in "in-vivo" single-dose studies. A single-dose study was conducted in 37 healthy volunteers using a pharmaceutical composition according to Example 1.

The reference product was a 60 mg tablet (Evista) that consists of Raloxifene HCL, anhydrous lactose, camauba wax, crospovidone, hypromellose, lactose monohydrate, Mg stearate, modified pharmaceutical glaze, PEG, polysorbate 80, PVP, propylene glycol and titanium dioxide (product B).

In the pharmacokinetic analysis of composition 1 according to Example 1, Raloxifene and its two metabolites Raloxifene-4'-glucorunide and Raloxifene-6'-glucorunide were measured separately. Table 3 shows the main pharmacokinetic parameters obtained from the studies.

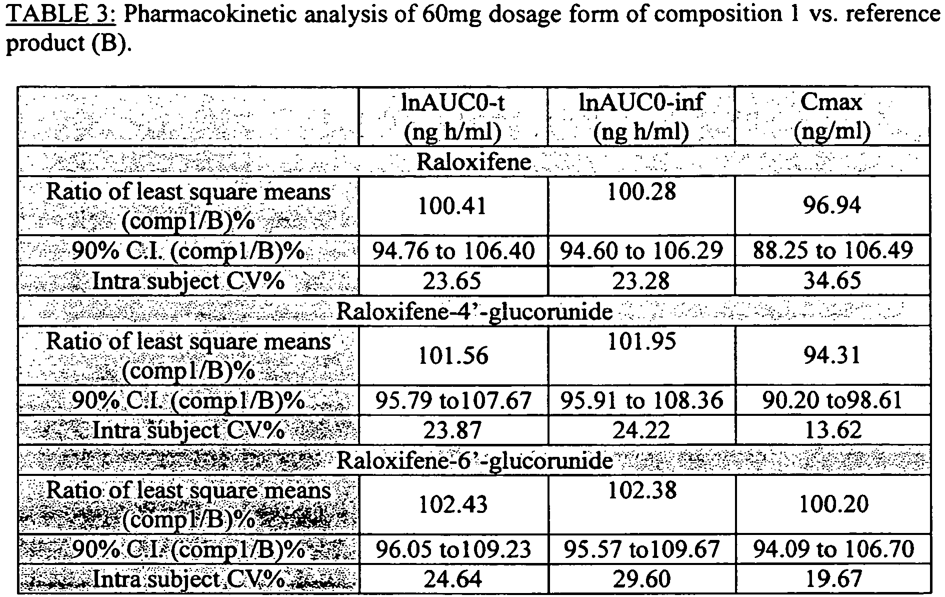

wherein:
C max= (peak concentration) is the highest concentration reached by the drug in plasma after dosing;
AUC₀₋ₜ = (area under the curve) the area under the plasma concentration versus time curve, from time 0 to the last measurable concentration, as calculated by the linear trapezoidal method.
AUC_{0-inf} = (area under the curve) the area under the plasma concentration versus time curve from time 0 to infinity. AUC inf is calculated as the sum of AUC 0-t plus the ratio of the last measurable plasma concentration to the elimination rate constant.

Clinical trial results demonstrate that the composition manufactured according to the present invention is bioavailable.

## Claims

1. A pharmaceutical composition for oral administration comprising selective estrogen receptor modulator Raloxifene hydrochloride as the active ingredient, an effective amount of super disintegrant sodium starch glycolate as a dissolution enhancing agent to facilitate the drug release and increase bioavailability, and pH adjusting agent citric acid monohydrate.

2. The pharmaceutical composition according to claim 1, wherein it comprises from about 0.5 to 30% by weight of Raloxifene hydrochloride and from about 3 to 30 % by weight of said sodium starch glycolate.

3. The pharmaceutical composition according to claim 2, wherein the weight ratio of said selective estrogen receptor modulator such as Raloxifene hydrochlorideto sodium starch glycolate is preferably 10:1 to 1:60.

4. The pharmaceutical composition according to claim 1, wherein it comprises approximately 0.5% to 30%, preferably 0.75% to 30% and most preferably 1% to 30% by weight of said selective estrogen receptor modulator Raloxifene hydrochloride.

5. The pharmaceutical composition according to claim 2, wherein it comprises approximately 3% to 30%, more preferably 5% to 25% and most preferably 7% to 20% by weight of sodium starch glycolate.

6. The pharmaceutical composition according to any preceding claim, wherein it further comprises at least one optionally excipient selected from the group consisting of diluents, binders, disintegrants, lubricants, and glidants.

7. The pharmaceutical composition according to any preceding claim, wherein said composition is in a solid dosage form such as a tablet, capsule or sachet comprising Raloxifene hydrochloride as active ingredient.

8. A process for the preparation of a solid dosage form for oral administration, such as a tablet, a capsule or a sachet, containing selective estrogen receptor modulator Raloxifene hydrochloride as active ingredient according to claims 1-7, which comprises:
- Forming a first homogenous blend by mixing the total quantity of said active ingredient with a total quantity of super disintegrant sodium starch glycolate as a dissolution enhancing agent to facilitate the drug release and increase bioavailability, and pH adjusting agent citric acid monohydrate;
- dissolving the total quantity of a surfactant such as Poloxamer 407 into purified water;
- adding the first blend into the formed solution;
- adding to the above kneaded mixture the total quantity of at least one optional excipient such as a binder, a disintegrant, a lubricant, a colorant and/or a glidant until uniform and wet granulating;
- drying the wet mass;
- sieving the dried material to achieve the desired granule size
- adding and mixing the dried material with at least one optional excipient of the external phase such as a binder, a disintegrant, a lubricant, a colorant and/or a glidant until uniform, and
- formulating the resulting mixture in a solid dosage form either by compressing it into a desired tablet form or by filling capsules or sachets.

## Patentansprüche

1. Pharmazeutische Zusammensetzung zur oralen Verabreichung, umfassend der selektive Östrogen-Rezeptor-Modulator Raloxifen-Hydrochlorid als Wirkstoff, einer wirksamen Menge des Super-Sprengmittel Natriumstärkeglycolat als die Auflösung verbesserndes Mittel, um die Arzneimittelfreisetzung und die Bioverfügbarkeit zu erleichtern und der pH-Einstellmittel Zitronensäure-Monohydrat.

2. Pharmazeutische Zusammensetzung nach Anspruch 1, wobei die von etwa 0,5% bis 30% nach Gewicht von Raloxifen-Hydrochlorid und von etwa 3 bis 30% nach Gewicht der vorgenannten Natriumstärkeglycolat umfasst.

3. Pharmazeutische Zusammensetzung nach Anspruch 2, wobei das Gewichtsverhältnis den selektiven Östrogen-Rezeptor-Modulator, wie Raloxifen-Hydrochlorid zu Natriumstärkeglycolat ist vorzugsweise 10:1 bis 1:60.

4. Pharmazeutische Zusammensetzung nach Anspruch 1, wobei die ungefähr 0,5% bis 30%, mehr bevorzugt 0,75% bis 30% und am meisten bevorzugt 1% bis 30% nach Gewicht der selektiven Östrogen-Rezeptor-Modulator Raloxifen-Hydrochlorid umfasst.

5. Pharmazeutische Zusammensetzung nach Anspruch 2, wobei die ungefähr 3% bis 30%, mehr bevorzugt 5% bis 25% und am meisten bevorzugt 7% bis 20% Gewichts Natriumstärkeglycolat umfasst.

6. Pharmazeutische Zusammensetzung nach einem der vorhergehenden Ansprüche, wobei die ferner mindestens einen Hilfsstoff gegebenenfalls ausgewählt, der aus der Gruppe von Verdünnungsmitteln, Bindemitteln, Sprengmitteln, Schmiermitteln und Gleitmitteln besteht.

7. Pharmazeutische Zusammensetzung nach einem der vorhergehenden Ansprüche, wobei die Zusammensetzung in einer festen Dosierungsform wie einer Tablette, Kapsel oder ein Beutel, enthaltend Raloxifen-Hydrochlorid als Wirkstoff ist.

8. Verfahren zur Herstellung einer festen Dosierungsform zur oralen Verabreichung, wie einer Tablette, einer Kapsel oder eines Beutels, der selektive Östrogen-Rezeptor-Modulator Raloxifen-Hydrochlorid als Wirkstoff (nach den Ansprüchen 1-7) enthaltend, der umfasst:
- Bildung einer ersten homogenen Mischung durch Mischen der Gesamtmenge des Wirkstoffs mit einer Gesamtmenge von der Super-Sprengmittel Natriumstärkeglycolat als die Auflösung verbesserndes Mittel, um die Arzneistofffreisetzung und die Erhöhung der Bioverfügbarkeit, und der pH-Einstellmittel Zitronensäure Monohydrat zu erleichtern;
- Auflösen der Gesamtmenge eines Tensids, wie Poloxamer 407 in gereinigtes Wasser;
- Hinzufügen der ersten Mischung zu der gebildeten Lösung;
- Hinzufügen die Gesamtmenge des mindestens einen Hilfsstoffe, wie einem Bindemittel, einem Sprengmittel, einem Schmiermittel, einem Farbemittel und / oder einem Gleitmittel zu der obigen geknetet Mischung, bis es homogen ist und Naßgranulieren;
- Trocknen der feuchten Masse;
- Sieben des getrockneten Materials die gewünschte Korngröße zu erreichen
- Hinzufügren und mischen des getrockneten Materials mit mindestens einem optionalen Hilfsstoff der äußeren Phase, wie ein Bindemittel, Sprengmittel, Gleitmittel, Farbmittel und / oder ein Fließregulierungsmittel, bis es homogen ist und
- Formulierung der resultierenden Mischung in eine feste Dosierungsform entweder durch Komprimieren in die gewünschte Tablettenform oder durch Füllen von Kapseln oder Beuteln.

## Revendications

1. Une composition pharmaceutique pour administration orale comprenant le modulateur sélectif de récepteur d'oestrogène: le chlorhydrate de Raloxifène comme principe actif, une quantité efficace de super-désintégrant: le glycolate d'amidon sodique comme agent améliorant la dissolution afin de faciliter la libération du médicament et augmenter sa biodisponibilité, et un agent d'ajustement de pH: l'acide citrique monohydraté.

2. La composition pharmaceutique selon la revendication 1, dans laquelle est compris environ de 0.5 à 30% en poids de chlorhydrate de Raloxifène et environ de 3 à 30% en poids du dit glycolate d'amidon sodique.

3. La composition pharmaceutique selon la revendication 2, dans laquelle le rapport pondéral du dit modulateur sélectif de récepteur d'oestrogène comme le chlorhydrate de Raloxifène et du glycolate d'amidon sodique est préférablement de 10:1 à 1:60.

4. La composition pharmaceutique selon la revendication 1, qui comprend approximativement de 0.5% à 30%, de préférence de 0.75% à 30%, et plus préférablement de 1% à 30% en poids du dit modulateur sélectif de récepteur d'oestrogène: le chlorhydrate de Raloxifène.

5. La composition pharmaceutique selon la revendication 2, qui comprend approximativement de 3% à 30%, de préférence de 5% à 25% et plus préférablement de 7% à 20% en poids de glycolate d'amidon sodique.

6. La composition pharmaceutique selon l'une quelconque des revendications précédentes, dans laquelle est compris en outre, facultativement, au moins un excipient choisi parmi le groupe constitué de diluants, liants, désintégrants, lubrifiants et d'agents de glissement.

7. La composition pharmaceutique selon l'une quelconque des revendications précédentes, dans laquelle la dite composition est sous forme galénique solide telle qu'un comprime, une gélule, ou un sachet.

8. Un procédé pour la préparation d'une forme galénique solide pour administration orale, telle qu'un comprimé, une gélule ou un sachet, contenant un modulateur sélectif de récepteur d'oestrogène: le chlorhydrate de Raloxifène comme principe actif, selon les revendications 1-7, qui comprend:
- former un premier mélange homogène en mélangeant la quantité totale du dit principe actif avec la quantité totale de super-désintégrant: glycolate d'amidon sodique comme agent améliorant la dissolution afin de faciliter la libération du médicament et augmenter la biodisponibilité, et l'agent d'ajustement du pH: acide citrique monohydrate;
- dissoudre la quantité totale d'un surfactant, tel que le Poloxamer 407 dans de l'eau purifiée;
- ajouter le premier mélange à la solution formée;
- ajouter au mélange malaxé ci-dessus, la quantité totale d'au moins un excipient facultatif, tel qu'un liant, un désintégrant, un lubrifiant, un colorant et/ou un agent de glissement jusqu'à uniformité et granuler par voie humide;
- sécher la masse humide;
- tamiser le matériau séché pour obtenir la taille de granule désirée ;
- ajouter et mélanger le matériau séché avec au moins un excipient facultatif de la phase externe, tel qu'un liant, un désintégrant, un lubrifiant, un colorant et/ou un agent de glissement jusqu'à uniformité; et
- formuler le mélange obtenu sous forme galénique solide soit en le comprimant sous la forme de comprimé désirée, soit en remplissant gélules ou sachets.
